# EUROPEAN PATENT APPLICATION

(11) **EP 4 647 445 A1**
(43) Date of publication of application: **12.11.2025**
(21) Application number: 23910566.1
(22) Date of filing: 26.12.2023
(51) Int. Cl.: C07K 16/28, C07K 16/40, C12N 15/13, A61K 39/395, A61P 35/00, A61P 35/02, G01N 33/573, G01N 33/68

(54) **ANTIGEN-BINDING PROTEINS TARGETING ROR1**

(30) Priority: 27.12.2022 CN 202211683175; 27.12.2022 CN 202211683173
(71) Applicant: SPH Biotherapeutics (Shanghai) Limited, Shanghai 200072 (CN); SPH Biotherapeutics (HK) Limited, New Territories Hong Kong 999077 (HK)
(72) Inventor: HUA, Jian, Shanghai 200072 (CN); ZHANG, Hua, Hong Kong 999077 (CN); ZHOU, Biao, Hong Kong 999077 (CN); CUI, Yuzhu, Hong Kong 999077 (CN); DENG, Linyan, Hong Kong 999077 (CN); WAN, Yuhua, Shanghai 200072 (CN); XU, Feng, Hong Kong 999077 (CN)
(74) Representative: karo IP
(86) International application number: PCT/CN2023/141794
(87) International publication number: WO 2024/140641

(57) **Abstract**

Provided are an isolated antigen-binding protein, a preparation method therefor, and use thereof. The isolated antigen-binding protein can bind to a receptor tyrosine kinase-like orphan receptor 1 (ROR1). The antigen protein comprises CDR1, CDR2, and CDR3.

## Description

### Field of the Invention

The present application relates to the field of biomedicine, and particularly to an antigen-binding protein targeting ROR1.

### Background of the Invention

ROR1 is a transmembrane receptor tyrosine kinase protein, and a human ROR1 is composed of one extracellular immunoglobulin-like domain (Ig), two cysteine-rich domains (FZD), a juxtamembrane kringle domain, a single-pass transmembrane structure and one intracellular tyrosine kinase domain (TKD), two serine/threonine-rich domains (S/TRD), and one proline-rich domain (PRD). ROR1 regulates cell division, proliferation, migration and chemotaxis by mediating the signal transduction in multiple signaling pathways. ROR1 is highly expressed during the early embryonic development. Along with the process of fetal development, the expression level of ROR1 gradually decreases.

ROR1 is lowly expressed or not expressed in normal human tissues, but highly expressed in various blood tumors and solid tumors. Blood tumors with high expression of ROR1 comprise B-cell chronic lymphocytic leukemia (CLL), acute lymphoblastic leukemia (ALL), non-Hodgkin lymphoma (NHL), and myeloid blood cancers. Among solid tumors, tumors with expression of ROR1 comprise triple-negative breast cancer, colon cancer, lung cancer, pancreatic cancer, ovarian cancer, etc., and the expression of ROR1 is also closely related to the progression of the disease and the therapeutic effect. Therefore, ROR1 can serve as a specific tumor marker and a potentially attractive target for the treatment of tumors.

### Summary of the Invention

The present application provides an isolated antigen-binding protein targeting ROR1. In the present application, the isolated antigen-binding protein is capable of specifically binding to an ROR1 antigen, and has a good binding activity. The isolated antigen-binding protein of the present application is capable of binding to an ROR1 expressed on the surface of a cell (e.g., A549 cells, MDA MB231 cells, MEC-ROR1 cells, JeKo-1 cells). The isolated antigen-binding protein of the present application can have a tumor-killing effect.

In one aspect, the present application provides an isolated antigen-binding protein capable of binding to a receptor tyrosine kinase-like orphan receptor 1 (ROR1), the isolated antigen protein comprises a CDR1, a CDR2, and a CDR3, the amino acid sequence of the CDR1 is as set forth in SEQ ID NO: 1, the amino acid sequence of the CDR2 is as set forth in SEQ ID NO: 2, and the amino acid sequence of the CDR3 is as set forth in SEQ ID NO: 3; or, the amino acid sequence of the CDR1 is as set forth in SEQ ID NO: 1, the amino acid sequence of the CDR2 is as set forth in SEQ ID NO: 2, and the amino acid sequence of the CDR3 is as set forth in SEQ ID NO: 13; or, the amino acid sequence of the CDR1 is as set forth in SEQ ID NO: 19, the amino acid sequence of the CDR2 is as set forth in SEQ ID NO: 20, and the amino acid sequence of the CDR3 is as set forth in SEQ ID NO: 21.

In some embodiments, the isolated antigen-binding protein is a single-domain antibody.

In some embodiments, the isolated antigen-binding protein comprises a VHH.

In some embodiments, the amino acid sequence of the VHH is as set forth in SEQ ID NO: 8, SEQ ID NO: 18 or SEQ ID NO: 25.

In some embodiments, the isolated antigen-binding protein comprises an antibody or an antigen-binding fragment thereof.

In some embodiments, the antibody is selected from a group consisting of: a monoclonal antibody, a polyclonal antibody, a chimeric antibody, a humanized antibody, and a fully human antibody.

In some embodiments, the isolated antigen-binding protein comprises at least one VHH fragment.

In some embodiments, the isolated antigen-binding protein comprises two VHH fragments.

In some embodiments, the isolated antigen-binding protein also comprises an Fc region.

In some embodiments, the Fc region is derived from an IgG Fc region.

In some embodiments, the Fc region is derived from a human IgG Fc region.

In some embodiments, the isolated antigen-binding protein has one or more of the following properties:
(1) capable of specifically binding to an ROR1; and
(2) capable of binding to an ROR1 expressed on a cell surface.

In another aspect, the present application further provides a chimeric antigen receptor, which comprises the isolated antigen-binding protein of the present application.

In some embodiments, the chimeric antigen receptor also comprises a transmembrane domain, a costimulatory domain, and an intracellular signaling domain.

In some embodiments, the transmembrane domain comprises a transmembrane domain derived from a protein selected from a group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154 or SLAM.

In some embodiments, the transmembrane domain is derived from a transmembrane domain of CD28, and the transmembrane domain comprises the amino acid sequence as set forth in SEQ ID NO: 28.

In some embodiments, the costimulatory domain comprises a costimulatory domain derived from one or more proteins selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

In some embodiments, the costimulatory domain is derived from a 4-1BB costimulatory domain, and the costimulatory domain comprises the amino acid sequence as set forth in SEQ ID NO: 29.

In some embodiments, the intracellular signaling domain comprises an intracellular signaling region derived from one or more proteins selected from a group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV)LMP2A, simian immunodeficiency virus PBj14 Nef, Kaposi's sarcoma-associated herpes virus (HSKV), DAP10, DAP-12, and a domain containing at least one ITAM.

In some embodiments, the intracellular signaling domain is a signaling domain derived from CD3ζ, and the intracellular signaling domain comprises the amino acid sequence as set forth in SEQ ID NO: 30.

In some embodiments, the chimeric antigen receptor also comprises a hinge region.

In some embodiments, the hinge region is derived from a CD28 hinge region.

In some embodiments, the hinge region comprises the amino acid sequence as set forth in SEQ ID NO: 27.

In some embodiments, the chimeric antigen receptor comprises the amino acid sequence as set forth in SEQ ID NO: 31.

In another aspect, the present application further provides an immune cell, which comprises the chimeric antigen receptor of the present application.

In some embodiments, the immune cell is selected from: a T cell, an NK cell, an iNKT cell, a CIK cell or a γδT cell.

In another aspect, the present application further provides a drug molecule, which comprises the isolated antigen-binding protein or the chimeric antigen receptor.

In another aspect, the present application further provides a nucleic acid molecule, which encodes the isolated antigen-binding protein or the chimeric antigen receptor.

In another aspect, the present application further provides a vector, which comprises the nucleic acid molecule.

In another aspect, the present application further provides a cell, which comprises the nucleic acid molecule and/or the vector.

In another aspect, the present application further provides a pharmaceutical composition, which comprises the isolated antigen-binding protein, the drug molecule, the immune cell, the nucleic acid molecule, the vector, and/or the cell, as well as optionally a pharmaceutically acceptable carrier.

In another aspect, the present application further provides a kit, which comprises the isolated antigen-binding protein or the chimeric antigen receptor, the kit being capable of detecting the presence and/or content of ROR1 in a sample.

In another aspect, the present application further provides use of the isolated antigen-binding protein, the drug molecule, the nucleic acid molecule, the vector, the cell and/or the pharmaceutical composition in the preparation of a drug for preventing and/or treating an ROR1-related disease and/or disorder.

In some embodiments, the disease and/or disorder comprises a tumor.

In some embodiments, the tumor comprises a solid tumor and/or a blood tumor.

In some embodiments, the tumor comprises an ROR1-positive tumor.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:
FIG. 1 shows the ROR1-specific antibody titers in pre- and post-immunization alpaca sera collected on days 1 to 5 after immunization as described in the present application.
FIG. 2 shows the size of the sequences amplified from the cDNA library as described in the present application. FIG. 2A shows that two different sets of amplicons were generated in the first PCR, with molecular weights of approximately 0.7 kb and 0.9 kb, respectively. FIG. 2B shows that a 0.7 kb amplicon was used as a template for amplification, resulting in a DNA fragment with a fragment size of approximately 400 bp.
FIG. 3 shows the plates used for calculating the number of colonies through a series of dilutions in the plates and thereby estimating the size of the library as described in the present application.
FIG. 4 shows the phylogenetic analysis diagram of 90 sequences in the library as described in the present application.
FIG. 5 shows the plates used for the continuous screening of three rounds of ROR1 specific antigen-binding proteins as described in the present application.
FIG. 6 shows the ELISA results of the present application, where FIG. 6A shows the OD450 values of the screened sequences 5-26, FIG. 6B shows the OD450 values of the screened sequences 4-92, and FIG. 6C shows the OD450 values of VHH1, VHH2, and anti-ROR1 mAb.
FIG. 7 shows the SDS-PAGE results of the purified antigen-binding proteins as described in the present application, where FIG. 7A shows the SDS-PAGE results of 5-26, and FIG. 7B shows the SDS-PAGE results of 4-92.
FIGs. 8A-8C show the results of the purified antigen-binding proteins as described in the present application specifically binding to a recombinant ROR1 protein, where FIG. 8A shows the results of the screened antigen-binding proteins 5-26, FIG. 8B shows the results of the screened antigen-binding proteins 4-92, and FIG. 8C shows the binding results of negative control and positive control.
FIGs. 9A-9B show the specificity verification results of the antigen-binding proteins as described in the present application, where FIG. 9A shows the results of 5-26, and FIG. 9B shows the results of 4-92.
FIGs. 10A-10B show the flow cytometry results of the antigen-binding proteins as described in the present application binding to A549 cells expressing ROR1, where FIG. 10A shows the results of 5-26, and FIG. 10B shows the results of 4-92.
FIGs. 11A-11B show the flow cytometry results of the antigen-binding proteins as described in the present application binding to MB231 cells expressing ROR1, where FIG. 11A shows the results of 5-26, and FIG. 11B shows the results of 4-92.
FIGs. 12A-12B show the flow cytometry results of the antigen-binding proteins as described in the present application binding to JeKo-1 cells expressing ROR1, where FIG. 12A shows the results of 5-26, and FIG. 12B shows the results of 4-92.
FIGs. 13A-13D show the flow cytometry results of a humanized ROR1 single-domain antibody binding to MEC cells, JeKo-1 cells, A549 cells and MB231 cells expressing ROR1; where FIG. 13A shows the flow cytometry results of binding to MEC cells expressing ROR1, FIG. 13B shows the flow cytometry results of binding to JeKo-1 cells expressing ROR1, FIG. 13C shows the flow cytometry results of binding to A549 cells expressing ROR1, and FIG. 13D shows the flow cytometry results of binding to MB231 cells expressing ROR1.
FIG. 14 shows the structural design of ROR1 nano-chimeric antigen receptor.
FIG. 15 shows that high level expression of CAR structure in activated T cells can be detected using ROR1 protein.
FIG. 16 shows the proportion of surviving target cells in the co-culture system after 24 hours when the initial effector-target ratio is 1:1.
FIG. 17 shows the secretion level of γ-interferon and IL2 in the supernatant of the co-culture system.
FIGs. 18A-18C show that the ROR1 nano-chimeric antigen receptor has a strong specific killing ability.

### Detailed Description of the Embodiments

The implementation of the present application will be illustrated in the following specific examples, and other advantages and effects of the present application will be easily known by those skilled in the art from the content disclosed in the specification.

### Definition of Terms

In the present application, the term "antigen-binding protein" generally refers to a protein containing an antigen-binding portion, and optionally a scaffold or backbone portion that allows the antigen-binding portion to adopt a conformation that facilitates the binding of the antigen-binding protein to the antigen. The antigen-binding protein may comprise, but not limited to, an antibody, an antigen-binding fragment (Fab, Fab', F(ab)₂, an Fv fragment, F(ab')₂, scFv, di-scFv, and/or dAb), an immunoconjugate, a multispecific antibody (e.g., a bispecific antibody), an antibody fragment, an antibody derivative, an antibody analogue, or a fusion protein, etc., as long as they exhibit the desired antigen binding activity. The "isolated antigen-binding protein" of the present application may comprise an antigen-binding portion, and optionally a scaffold or framework portion that allows the antigen-binding portion to adopt a conformation that facilitates the binding of the antigen binding portion to the antigen.

In the present application, the term "single-domain antibody" generally refers to a fragment containing a single variable domain in an antibody, which can selectively bind to a specific antigen. The single-domain antibody of the present application may comprise one heavy chain variable domain, the single-domain antibody of the present application may comprise an antigen-binding fragment, and the antigen-binding fragment may comprise a VHH.

In the present application, the term "polypeptide" generally refers to a polymer of amino acid residues. This term also applies to amino acid polymers in which one or more amino acid residues are analogues or mimics of corresponding naturally occurring amino acids, and to naturally occurring amino acid polymers. This term may also comprise amino acid polymers that are modified, for example, by the addition of sugar residues to form glycoproteins or by phosphorylation. The polypeptide may be produced from naturally occurring and non-recombinant cells or from genetically engineered or recombinant cells, and may comprise a molecule having the amino acid sequence of a natural protein, or a molecule having deletions, additions and/or substitutions of one or more amino acids of the natural sequence. The term "polypeptide" may comprise an antigen-binding fragment, an antibody or a sequence having deletions, additions and/or substitutions of one or more amino acids of the antigen-binding fragment.

In the present application, the term "nucleic acid" generally refers to a polymer of nucleotides (e.g., ribonucleotide or deoxyribonucleotide), and may comprise naturally occurring nucleic acids (adenine, guanine, cytosine, uracil, and thymidine), non-naturally occurring nucleic acids, and modified nucleic acids. The term "nucleic acid" may comprise a gene, cDNA or mRNA. For example, the nucleic acid molecule may be synthesized (e.g., chemically synthesized) or recombinant. The nucleic acid may comprise a nucleic acid containing analogs or derivatives of naturally occurring nucleotides, which has similar binding properties to those of the reference nucleic acid and is metabolized in a manner similar to naturally occurring nucleotides. The nucleic acid sequence may also comprise conservative modified variants thereof (for example, degenerate codon substituted), alleles, orthologs, SNPs, and complementary sequences as well as the sequences explicitly indicated. The term is not limited by the length of the polymer. The nucleic acid may be single or double stranded and generally contain 5'-3' phosphodiester bonds, and nucleotide analogs may have other linkages.

In the present application, the term "constant region" generally refers to the sum of the domains of an antibody other than the variable region. The constant region does not directly involve the binding of antigens, but exhibits various effector functions. Depending on the amino acid sequences of the constant regions of heavy chains thereof, antibodies are divided into the following categories: IgA, IgD, IgE, IgG, and IgM, and some of these can be further divided into categories such as IgG1, IgG2, IgG3 and IgG4, IgA1 and IgA2. The constant regions of heavy chains corresponding to different types of antibodies are respectively referred to as α, δ, ε, γ, and µ.

In the present application, the term "antigen-binding fragment" generally refers to a polypeptide fragment of an immunoglobulin or an antibody that binds to an antigen or competes with an intact antibody (i.e., the intact antibody from which it is derived from) for antigen binding (i.e., specific binding). The antigen-binding fragment may comprise, but not limited to: Fab, Fab', F (ab')2, and an Fv fragment, VHH, a linear antibody, a single-chain antibody, a diabody, and a multispecific antibody composed of antibody fragments.

In the present application, the term "variable region" or "variable domain" generally refers to a part of the light chain and/or the heavy chain of an antibody, which generally comprises about 120-130 amino-terminal amino acids in the heavy chain and about 100-110 amino-terminal amino acids in the light chain. Variable regions are generally widely different in amino acid sequences, and even in antibodies of the same species. The variable region of an antibody may determine the binding and specificity of each specific antibody for its specific antigen. The variability of a sequence is concentrated in regions called complementary determining regions (CDRs), while the regions with higher conservation among the variable regions are called framework regions (FRs). The CDRs of the light and heavy chains may contain amino acids that are largely responsible for the direct interaction between antibodies and antigens.

In the present application, the term "chimeric antigen receptor (CAR)" generally refers to a recombinant polypeptide comprising at least an extracellular domain, a transmembrane region, and an intracellular domain that specifically bind to an antigen or a target. For example, a hinge region is comprised between the extracellular domain and the transmembrane region. For example, the chimeric antigen receptor may comprise a signal peptide. The binding of the extracellular domain of CAR to the target antigen on the surface of target cells leads to CAR clustering and delivery of activation stimulus to the CAR-containing cells. CAR redirects the specificity of immune effector cells, and triggers proliferation, cytokine production, and phagocytosis and/or production of molecules capable of mediating the death of cells expressing target antigens in a manner that is not dependent on major histocompatibility (MHC).

In the present application, the term "intracellular domain" refers to an intracellular domain comprising any truncated portions that are sufficient to transduce the activation signals. The intracellular domain may comprise an intracellular signaling region and/or a costimulatory signaling region. The term "intracellular signaling region" refers to an intracellular region that can produce a signal that promotes the immune effector function of CAR-containing cells (e.g., CART cells or CAR-expressing NK cells). For example, the intracellular signaling region may comprise an intracellular signaling region of one or more proteins selected from a group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj 14 Nef, Kaposi's sarcoma-associated herpes virus (HSKV), DAP10, DAP-12, and a domain containing at least one ITAM. For example, the intracellular signaling region may be a signaling domain derived from CD3ζ. The term "costimulatory signaling region" refers to a portion of CAR in the intracellular signaling domain that can transduce effector signals. For example, the costimulatory signaling region may comprise an intracellular costimulatory signaling region derived from one or more proteins selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88. For example, the costimulatory signaling region may be an intracellular costimulatory signaling region derived from 4-1BB.

In the present application, the term "transmembrane domain" generally refers to a domain of a peptide, a polypeptide or a protein capable of spanning the cytoplasmic membrane. These domains can be used to anchor an extracellular domain on the cell membrane. For example, the transmembrane region may comprise a transmembrane domain of one or more proteins selected from a group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM. For example, the transmembrane domain may be derived from a transmembrane region of CD8.

In the present application, the term "signal peptide" generally refers to a leader sequence at the amino terminus (N-terminus) of a nascent CAR protein which directs the nascent protein to the endoplasmic reticulum during or after translation, followed by surface expression. For example, the signal peptide is derived from a signal peptide of CD8 protein.

In the present application, the term "drug molecule" generally may comprise any drug forms known in the art. For example, the drug molecule may be protein. For example, the drug molecule may be polypeptide. For example, the drug molecule may be conjugates of drugs in different forms. For example, the drug molecule may comprise small molecular drugs.

In the present application, the term "immune effector cell" generally refers to immune cells that participate in immune responses and perform effector functions. For example, the exercise of effector functions may comprise removal of foreign antigens or promotion of immune effector responses, etc. For example, the immune effector cell may comprise T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells. For example, the immune effector cell may be a T cell.

In the present application, the term "host cell" generally refers to an individual cell, cell line, or cell culture that may comprise or has comprised a plasmid or vector containing the nucleic acid molecule of the present application, or that can express the fusion protein of the present application or the antigen-binding fragment thereof. The cell may comprise the progeny of a single cell. Due to natural, accidental, or intentional mutations, the progeny cells may not necessarily be exactly the same as the original parent cells in terms of morphology or genome, as long as they can express the antigen-binding protein of the present application. The cells can be obtained by transfecting cells with the vector of the present application *in vitro.* The cells may be prokaryotic cells (e.g., *E. coli*) or eukaryotic cells (e.g., yeast cells, e.g., COS cells, Chinese Hamster Ovary (CHO) cells, HeLa cells, HEK293 cells, COS-1 cells, NS0 cells, or myeloma cells).

In the present application, the term "vector" generally refers to a nucleic acid molecule that is capable of self-replicating in a suitable host and that transfers the inserted nucleic acid molecule into and/or between cells (e.g., host cells). The vector may comprise vectors primarily for inserting DNA or RNA into cells, vectors primarily for replicating DNA or RNA, as well as vectors primarily for expression by transcription and/or translation of DNA or RNA. The vector also comprises vectors having a plurality of the above functions. The vector may be a polynucleotide that can be transcribed and translated into a polypeptide when introduced into a suitable cell. Generally, the vector can produce the desired expression product by culturing suitable cells containing the vector. In the present application, the vector may be a plasmid.

In the present application, the term "nucleic acid molecules" comprises DNA molecules and RNA molecules. The nucleic acid molecule may be single- or double-stranded. The term "promoter" generally refers to a DNA sequence that can regulate the expression of a selected DNA sequence operatively linked to the promoter, thereby affecting the expression of the selected DNA sequence in the cell. For example, the nucleic acid molecule may encode the antigen-binding protein and/or the chimeric antigen receptor. For example, the nucleic acid molecule may comprise a promoter. For example, the promoter may be a constitutive promoter.

In the present application, the term "pharmaceutically acceptable" generally refers to non-toxic materials that do not interfere with the effectiveness of the biological activity of the active ingredients. Such preparations may routinely contain pharmaceutically acceptable concentrations of salts, buffers, preservatives, compatible carriers, supplementary immunopotentiators such as adjuvants and cytokines, and optionally other therapeutic agents such as chemotherapeutic agents.

In the present application, the term "complementary determining region" or the term "CDR" generally refers to a complementary determining region in a variable sequence of an antibody. There are three CDRs in each variable region of a heavy chain and/or a light chain, which are named as CDR1, CDR2, and CDR3 for each variable region. As used herein, a CDR combination may refer to a group of three CDRs occurring in a single variable region that can bind to an antigen. The exact boundaries of these CDRs have been defined differently according to different systems. The system described by Kabat (Kabat et al, Sequences of Proteins of Immunological Interest National Institutes of Health, Bethesda, Md. (1987) and (1991)) not only provides a definite residue numbering system for any variable regions of an antibody, but also provides exact residue boundaries for defining the three CDRs. These CDRs can also be referred to as Kabat CDRs. Chothia and colleagues (Chothia and Lesk, J.Mol.Biol.196: 901-917 (1987) and Chothia et al, Nature 342: 877-883 (1989)) have found that certain subregions within the Kabat CDRs adopt almost the same peptide backbone conformation, despite exhibiting substantial diversity at the amino acid sequence level. These subregions are named as L1, L2, and L3 or H1, H2, and H3, where "L" and "H" refer to regions of light chain and heavy chain, respectively. These regions can be referred to as Chothia CDRs, which have overlapped boundaries with Kabat CDRs. Other boundaries for defining CDRs that are overlapped with Kabat CDRs have been described by Padlan (FASEB J.9: 133-139 (1995)) and MacCallum (J Mol Biol 262 (5): 732-45 (1996)). Other CDR boundary definitions may not strictly follow one of the above systems, but they will still overlap with Kabat CDRs, although they may be shortened or lengthened based on predictions or experimental findings that specific residues or residue groups or even the entire CDRs do not significantly affect antigen binding. The CDRs herein can be defined using the IMGT classification mode.

In the present application, the term "pharmaceutical composition" generally refers to a composition suitable for being administered to patients that may be human patients. For example, the pharmaceutical composition of the present application may comprise the antigen-binding protein of the present application, the immunoconjugate of the present application, the nucleic acid molecule of the present application, the vector of the present application and/or the cell of the present application, as well as optionally a pharmaceutically acceptable carrier. In addition, the pharmaceutical composition may also comprise suitable preparations of one or more (pharmaceutically effective) carriers, stabilizers, excipients, diluents, solubilizers, surfactants, emulsifiers and/or preservatives. The acceptable ingredients of the composition may be non-toxic to the recipient at the dosage and concentration used. The pharmaceutical composition of the present application may comprise, but not limited to, liquid, frozen and freeze-dried compositions.

In the present application, the term "ROR1" generally refers to a receptor tyrosine kinase-like orphan receptor 1. In the present application, the term covers a full-length ROR1 and a functional active fragment or a variant thereof. For example, the term may comprise an Ig-like domain and/or a coiled domain of ROR1. For example, the term may comprise amino acids at positions 30-305 of ROR1. For example, the sequence of a full-length human ROR1 antigen can be recorded under the GeneBank accession number Q01973.

The protein of the present application may also comprise functional variants, derivatives, analogues, homologues, and fragments thereof.

The term "functional variant" refers to a polypeptide having substantially the same amino acid sequence or encoded by substantially the same nucleotide sequence as the naturally occurring sequence and capable of having one or more activities of the naturally occurring sequence. In the context of the present application, the variant of any given sequence refers to a sequence in which a particular sequence of residues (either amino acid or nucleotide residues) has been modified so that the polypeptide or polynucleotide maintains substantially at least one endogenous function. The variant sequence can be obtained through the addition, deletion, substitution, modification, replacement and/or variation of at least one amino acid residue and/or nucleotide residue present in a naturally occurring protein and/or polynucleotide, as long as the original functional activity is maintained.

In the present application, the term "derivative" generally refers to a polypeptide or polynucleotide of the present application comprising any substitution, variation, modification, replacement, deletion and/or addition from/to one (or more) amino acid residues of the sequence, provided that the resulting polypeptide or polynucleotide substantially maintains at least one endogenous function.

In the present application, the term "analogue" generally, with respect to a polypeptide or polynucleotide, comprises any mimetics of the polypeptide or polynucleotide, that is, a chemical compound having at least one endogenous function of the polypeptide or polynucleotide that the mimetic mimics.

In general, amino acids can be substituted, for example, at least 1 (e.g., 1, 2, 3, 4, 5, 6, 7, 8, 9, 10 or 20 or more) amino acids can be substituted, provided that the modified sequence substantially maintains the required activity or capability. Amino acid substitution may comprise the use of non-naturally occurring analogues.

The protein or polypeptide used in the present application may also have deletion, insertion or substitution of amino acid residues, where the amino acid residues undergo silent changes and result in functionally equivalent proteins. Intentional amino acid substitutions can be made based on the similarity of the polarity, charge, solubility, hydrophobicity, hydrophilicity, and/or amphoteric properties of the residues, as long as the endogenous function is maintained. For example, negatively charged amino acids comprise aspartic acid and glutamic acid; positively charged amino acids comprise lysine and arginine; and amino acids containing uncharged polar head-groups with a similar hydrophilic value comprise asparagine, glutamine, serine, threonine and tyrosine.

### Detailed Description of the Invention

### Isolated antigen-binding protein

In one aspect, the present application provides an isolated antigen-binding protein capable of binding to ROR1. For example, the binding may be specific binding. In the present application, the isolated antigen-binding protein is capable of binding to ROR1, and can be used in a variety of fields such as antibody drug development, bispecific antibody drug development, chimeric antigen receptor development, and the like.

In the present application, the isolated antigen-binding protein may comprise at least one CDR in a heavy chain variable region VH of an antibody, and the CDR may be an HCDR1, an HCDR2, and/or an HCDR3. For example, the heavy chain variable region VH may comprise the amino acid sequence as set forth in SEQ ID NO: 8, SEQ ID NO: 18 or SEQ ID NO: 25. In the present application, the HCDR sequence of the isolated antigen-binding protein may comprise HCDR sequences obtained by division in any form, provided that the VH sequence is identical to the amino acid sequence as set forth in SEQ ID NO: 8, SEQ ID NO: 18 or SEQ ID NO: 25, and the HCDR sequences obtained by division in any form fall within the scope of protection of the present application. For example, CDRs are divided by means of CCG, Kabat, Chothia, IMGT, and the combination of Kabat/Chothia. In some specific embodiments, CDRs can be divided using IMGT.

In the present application, the isolated antigen-binding protein may comprise an HCDR3, and the HCDR3 may comprise the amino acid sequence as set forth in SEQ ID NO: 3, SEQ ID NO: 13 or SEQ ID NO: 21.

In the present application, the isolated antigen-binding protein may comprise an HCDR2, and the HCDR2 may comprise the amino acid sequence as set forth in SEQ ID NO: 2 or SEQ ID NO: 20.

In the present application, the isolated antigen-binding protein may comprise an HCDR1, and the HCDR1 may comprise the amino acid sequence as set forth in SEQ ID NO: 1 or SEQ ID NO: 19.

In the present application, the isolated antigen-binding protein may comprise an HCDR1, an HCDR2, and an HCDR3. For example, the HCDR1 may comprise the amino acid sequence as set forth in SEQ ID NO: 1, the HCDR2 may comprise the amino acid sequence as set forth in SEQ ID NO: 2, and the HCDR3 may comprise the amino acid sequence as set forth in SEQ ID NO: 3. For example, the HCDR1 may comprise the amino acid sequence as set forth in SEQ ID NO: 1, the HCDR2 may comprise the amino acid sequence as set forth in SEQ ID NO: 2, and the HCDR3 may comprise the amino acid sequence as set forth in SEQ ID NO: 13. For example, the HCDR1 may comprise the amino acid sequence as set forth in SEQ ID NO: 19, the HCDR2 may comprise the amino acid sequence as set forth in SEQ ID NO: 20, and the HCDR3 may comprise the amino acid sequence as set forth in SEQ ID NO: 21.

In the present application, the VH of the isolated antigen-binding protein may comprise framework regions H-FR1, H-FR2, H-FR3, and/or H-FR4.

In the present application, the isolated antigen-binding protein may comprise an H-FR1, a C-terminus of the H-FR1 may be linked to an N-terminus of the HCDR1 directly or indirectly, and the H-FR1 may comprise the amino acid sequence as set forth in SEQ ID NO: 4 or SEQ ID NO: 14.

In the present application, the isolated antigen-binding protein may comprise an H-FR2, the H-FR2 may be located between the HCDR1 and the HCDR2, and the H-FR2 may comprise the amino acid sequence as set forth in SEQ ID NO: 5, SEQ ID NO: 15 or SEQ ID NO: 22.

In the present application, the isolated antigen-binding protein may comprise an H-FR3, the H-FR3 may be located between the HCDR2 and the HCDR3, and the H-FR3 may comprise the amino acid sequence as set forth in SEQ ID NO: 6, SEQ ID NO: 16 or SEQ ID NO: 23.

In the present application, the isolated antigen-binding protein may comprise an H-FR4, an N-terminus of the H-FR4 may be linked to a C-terminus of the HCDR3 directly or indirectly, and the H-FR4 may comprise the amino acid sequence as set forth in SEQ ID NO: 7, SEQ ID NO: 17 or SEQ ID NO: 24.

In the present application, the isolated antigen-binding protein may comprise an H-FR1, an H-FR2, an H-FR3, and an H-FR4. For example, the H-FR1 may comprise the amino acid sequence as set forth in SEQ ID NO: 4, the H-FR2 may comprise the amino acid sequence as set forth in SEQ ID NO: 5, the H-FR3 may comprise the amino acid sequence as set forth in SEQ ID NO: 6, and the H-FR4 may comprise the amino acid sequence as set forth in SEQ ID NO: 7. For example, the H-FR1 may comprise the amino acid sequence as set forth in SEQ ID NO: 14, the H-FR2 may comprise the amino acid sequence as set forth in SEQ ID NO: 15, the H-FR3 may comprise the amino acid sequence as set forth in SEQ ID NO: 16, and the H-FR4 may comprise the amino acid sequence as set forth in SEQ ID NO: 17. For example, the H-FR1 may comprise the amino acid sequence as set forth in SEQ ID NO: 4, the H-FR2 may comprise the amino acid sequence as set forth in SEQ ID NO: 22, the H-FR3 may comprise the amino acid sequence as set forth in SEQ ID NO: 23, and the H-FR4 may comprise the amino acid sequence as set forth in SEQ ID NO: 24.

In the present application, the isolated antigen-binding protein may comprise an HCDR1, an HCDR2, an HCDR3, an H-FR1, an H-FR2, an H-FR3, and/or an H-FR4, and a C-terminus of the H-FR1 is linked to an N-terminus of the HCDR1 directly or indirectly, the H-FR2 is located between the HCDR1 and the HCDR2, the H-FR3 is located between the HCDR2 and the HCDR3, an N-terminus of the H-FR4 is linked to a C-terminus of the HCDR3 directly or indirectly. In the present application, direct or indirect linkage is through intermolecular forces or through a linker.

In the present application, the isolated antigen-binding protein may comprise a heavy chain variable region VH, and the VH may comprise the amino acid sequence as set forth in SEQ ID NO: 8, SEQ ID NO: 18 or SEQ ID NO: 25.

In the present application, the isolated antigen-binding protein may comprise a heavy chain constant region, and the heavy chain constant region may comprise an Fc fragment. The Fc region may be derived from an IgG Fc region, e.g., a human IgG Fc region; e.g., an IgG1 Fc region; e.g., an IgG4 Fc region. For example, the Fc fragment may comprise a variant thereof, and the variant may comprise that there are substitution, deletion and/or addition of one or more amino acids in the amino acid sequence of the Fc fragment, for example, substitution, deletion and/or insertion of 1-30, 1-20 or 1-10, further for example, 1, 2, 3, 4, 5, 6, 7, 8 or 9 amino acids; alternatively, the Fc fragment may encompass a homologue thereof, and the homologue may be amino acid sequences having at least about 85% (e.g., at least about 85%, about 90%, about 91%, about 92%, about 93%, about 94%, about 95%, about 96%, about 97%, about 98%, about 99% or higher) sequence homology with the amino acid sequence of the Fc fragment.

In the present application, the isolated antigen-binding protein may comprise an antibody or an antigen-binding fragment thereof.

In some embodiments, the antigen-binding fragment in the isolated antigen-binding protein may be selected from a group consisting of: Fab, Fab', F (ab)2, an Fv fragment, F (ab')2, scFv, di-scFv, VHH, and/or dAb.

In the present application, the antibody in the isolated antigen-binding protein may be selected from a group consisting of: a monoclonal antibody, a single-chain antibody, a chimeric antibody, a humanized antibody, and a fully human antibody.

### Single-domain antibody

In the present application, the isolated antigen-binding protein may be a single-domain antibody.

In the present application, the single-domain antibody may comprise at least one CDR in the heavy chain variable region VHH of an antibody, and the CDR may be CDR3, CDR2, and/or CDR1. The heavy chain variable region VHH may comprise the amino acid sequence as set forth in SEQ ID NO: 8, SEQ ID NO: 18 or SEQ ID NO: 25.

In the present application, the single-domain antibody may comprise a CDR3, and the CDR3 may comprise the amino acid sequence as set forth in SEQ ID NO: 3, SEQ ID NO: 13 or SEQ ID NO: 21.

In the present application, the single-domain antibody may comprise a CDR2, and the CDR2 may comprise the amino acid sequence as set forth in SEQ ID NO: 2 or SEQ ID NO: 20.

In the present application, the single-domain antibody may comprise a CDR1, and the CDR1 may comprise the amino acid sequence as set forth in SEQ ID NO: 1 or SEQ ID NO: 19.

In the present application, the single-domain antibody may comprise a CDR1, a CDR2, and a CDR3. For example, the CDR1 may comprise the amino acid sequence as set forth in SEQ ID NO: 1, the CDR2 may comprise the amino acid sequence as set forth in SEQ ID NO: 2, and the CDR3 may comprise the amino acid sequence as set forth in SEQ ID NO: 3. For example, the CDR1 may comprise the amino acid sequence as set forth in SEQ ID NO: 1, the CDR2 may comprise the amino acid sequence as set forth in SEQ ID NO: 2, and the CDR3 may comprise the amino acid sequence as set forth in SEQ ID NO: 13. For example, the CDR1 may comprise the amino acid sequence as set forth in SEQ ID NO: 19, the CDR2 may comprise the amino acid sequence as set forth in SEQ ID NO: 20, and the CDR3 may comprise the amino acid sequence as set forth in SEQ ID NO: 21.

In the present application, the single-domain antibody may comprise an FR1, a C-terminus of the FR1 may be linked to an N-terminus of the CDR1 directly or indirectly, and the FR1 may comprise the amino acid sequence as set forth in SEQ ID NO: 4 or SEQ ID NO: 14.

In the present application, the single-domain antibody may comprise an FR2, the FR2 may be located between the CDR1 and the CDR2, and the FR2 may comprise the amino acid sequence as set forth in SEQ ID NO: 5, SEQ ID NO: 15 or SEQ ID NO: 22.

In the present application, the single-domain antibody may comprise an FR3, the FR3 may be located between the CDR2 and the CDR3, and the FR3 may comprise the amino acid sequence as set forth in SEQ ID NO: 6, SEQ ID NO: 16 or SEQ ID NO: 23.

In the present application, the single-domain antibody may comprise an FR4, an N-terminus of the FR4 may be linked to a C-terminus of the CDR3 directly or indirectly, and the FR4 may comprise the amino acid sequence as set forth in SEQ ID NO: 7, SEQ ID NO: 17 or SEQ ID NO: 24.

In the present application, the single-domain antibody may comprise an FR1, an FR2, an FR3, and an FR4. For example, the FR1 may comprise the amino acid sequence as set forth in SEQ ID NO: 4, the FR2 may comprise the amino acid sequence as set forth in SEQ ID NO: 5, the FR3 may comprise the amino acid sequence as set forth in SEQ ID NO: 6, and the FR4 may comprise the amino acid sequence as set forth in SEQ ID NO: 7. For example, the FR1 may comprise the amino acid sequence as set forth in SEQ ID NO: 14, the FR2 may comprise the amino acid sequence as set forth in SEQ ID NO: 15, the FR3 may comprise the amino acid sequence as set forth in SEQ ID NO: 16, and the FR4 may comprise the amino acid sequence as set forth in SEQ ID NO: 17. For example, the FR1 may comprise the amino acid sequence as set forth in SEQ ID NO: 4, the FR2 may comprise the amino acid sequence as set forth in SEQ ID NO: 22, the FR3 may comprise the amino acid sequence as set forth in SEQ ID NO: 23, and the FR4 may comprise the amino acid sequence as set forth in SEQ ID NO: 24.

In the present application, the single-domain antibody may comprise a CDR1, a CDR2, a CDR3, an FR1, an FR2, an FR3, and/or an FR4, and a C-terminus of the FR1 is linked to an N-terminus of the CDR1 directly or indirectly, the FR2 is located between the CDR1 and the CDR2, the FR3 is located between the CDR2 and the CDR3, and an N-terminus of the FR4 is linked to a C-terminus of the CDR3 directly or indirectly. In the present application, direct or indirect linkage is through intermolecular forces or through a linker.

In the present application, the single-domain antibody may comprise a heavy chain variable region VHH, and the VHH may comprise the amino acid sequence as set forth in SEQ ID NO: 8, SEQ ID NO: 18 or SEQ ID NO: 25.

In the present application, the single-domain antibody may comprise a heavy chain constant region, and the heavy chain constant region may comprise an Fc fragment. For example, the Fc fragment may comprise a variant thereof, and the variant may comprise that there are substitution, deletion and/or addition of one or more amino acids in the amino acid sequence of the Fc fragment, for example, substitution, deletion and/or insertion of 1-30, 1-20 or 1-10, further for example, 1, 2, 3, 4, 5, 6, 7, 8 or 9 amino acids; alternatively, the Fc fragment may encompass a homologue thereof, and the homologue may be amino acid sequences having at least about 85% (e.g., at least about 85%, about 90%, about 91%, about 92%, about 93%, about 94%, about 95%, about 96%, about 97%, about 98%, about 99% or higher) sequence homology with the amino acid sequence of the Fc fragment.

### Chimeric antigen receptor and Immune cell containing the chimeric antigen receptor

In another aspect, the present application provides a chimeric antigen receptor, which comprises an antigen-binding domain. For example, the antigen-binding domain may comprise the isolated antigen-binding protein of the present application.

In the present application, the antigen-binding domain of the chimeric antigen receptor may comprise CDR1, CDR2 and CDR3 of an antibody. For example, the CDR1 comprises the amino acid sequence as set forth in SEQ ID NO: 1, the CDR2 comprises the amino acid sequence as set forth in SEQ ID NO: 2, and the CDR3 comprises the amino acid sequence as set forth in SEQ ID NO: 3. For example, the CDR1 comprises the amino acid sequence as set forth in SEQ ID NO: 1, the CDR2 comprises the amino acid sequence as set forth in SEQ ID NO: 2, and the CDR3 comprises the amino acid sequence as set forth in SEQ ID NO: 13. For example, the CDR1 comprises the amino acid sequence as set forth in SEQ ID NO: 19, the CDR2 comprises the amino acid sequence as set forth in SEQ ID NO: 20, and the CDR3 comprises the amino acid sequence as set forth in SEQ ID NO: 21.

In the present application, the chimeric antigen receptor may comprise a costimulatory signaling region which can provide stimulatory signals. For example, the costimulatory signaling region may comprise an intracellular costimulatory signaling region of one or more proteins selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

In the present application, the costimulatory signaling region may be an intracellular costimulatory signaling region derived from 4-1BB. For example, the costimulatory signaling region may comprise the amino acid sequence as set forth in SEQ ID NO: 29. In the present application, the CAR may comprise an intracellular signaling domain which can transmit activation signals into the cells. For example, the intracellular signaling region may comprise an intracellular signaling region derived from one or more proteins selected from a group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj14 Nef, Kaposi's sarcoma-associated herpes virus (HSKV), DAP10, DAP-12, and other domains containing at least one ITAM.

For example, the intracellular signaling region may be a signaling domain derived from CD3ζ. For example, the intracellular signaling region may comprise the amino acid sequence as set forth in SEQ ID NO: 30.

In the present application, the CAR may comprise a transmembrane domain, and the transmembrane domain is a sequence in the cell surface protein that spans the cell membrane, for example, the sequence may comprise a hydrophobic alpha helix. The transmembrane domain may be derived from any type I transmembrane protein. The transmembrane domain may be predicted as a synthetic sequence for forming the hydrophobic helix. For example, the transmembrane domain may comprise a transmembrane domain derived from one or more proteins selected from a group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

For example, the transmembrane region may be a transmembrane domain derived from CD28. For example, the transmembrane domain may comprise the amino acid sequence as set forth in SEQ ID NO: 28.

In the present application, the chimeric antigen receptor may also comprise a hinge region, the hinge region may be located between the extracellular targeting portion and the transmembrane domain. For example, the hinge region may comprise a hinge region of one or more proteins selected from a group consisting of: CD28, IgG1, IgG4, IgD, 4-1BB, CD4, CD27, CD7, CD8, PD-1, ICOS, OX40, NKG2D, NKG2C, FcεRIγ, BTLA, GITR, DAP10, CD40L, TIM1, CD226, SLAM, CD30, and LIGHT.

In the present application, the hinge region may be a hinge region derived from CD28. For example, the hinge region may comprise the amino acid sequence as set forth in SEQ ID NO: 27.

In the present application, the chimeric antigen receptor may also comprise a signal peptide at the N-terminus of the binding domain that binds to the ROR1 protein. For example, the signal peptide may be a signal peptide derived from the CD8 protein. For example, the signal peptide may comprise the amino acid sequence as set forth in SEQ ID NO: 26.

In the present application, the chimeric antigen receptor may comprise the amino acid sequence as set forth in SEQ ID NO: 31.

In another aspect, the present application further provides an immune cell which comprises and/or expresses the chimeric antigen receptor of the present application. For example, the immune cell may comprise T cells, B cells, natural killer cells (NK cells), macrophages, NKT cells, monocytes, dendritic cells, granulocytes, lymphocytes, leukocytes, peripheral blood mononuclear cells, embryonic stem cells, lymphoid progenitor cells and/or pluripotent stem cells.

In some embodiments, the cell may be a T cell.

### Polypeptide, drug molecule, nucleic acid, vector, cell, preparation method

In another aspect, the present application provides a polypeptide which may comprise the isolated antigen-binding protein or the chimeric antigen receptor.

In another aspect, the present application provides a drug molecule which may comprise the isolated antigen-binding protein or the chimeric antigen receptor.

In another aspect, the present application provides one or more isolated nucleic acid molecules which may encode the isolated antigen-binding protein, the chimeric antigen receptor or the polypeptide of the present application. For example, each one nucleic acid molecule of the one or more nucleic acid molecules can encode the isolated antigen-binding protein, the chimeric antigen receptor, or the polypeptide as a whole, or encode a part thereof. The nucleic acid molecule of the present application may be isolated. In the present application, a variety of methods known in the art can be used to prepare the nucleic acid encoding the isolated antigen-binding protein, the chimeric antigen receptor or the polypeptide.

In another aspect, the present application provides one or more vectors, which comprise the one or more nucleic acid molecules of the present application. Each vector may comprise one or more of the nucleic acid molecules. In addition, the vector may further comprise other gene(s), e.g., a marker gene that allows the selection of the vector in an appropriate host cell and under appropriate conditions. In addition, the vector may contain various elements for controlling the expression, comprising a promoter sequence, a transcription initiation sequence, an enhancer sequence, a selective element and a reporter gene. In addition, the vector may also contain a replication initiation site. In addition, the vector may comprise, e.g., plasmid, cosmid, virus, phage, or other vectors commonly used in, e.g., genetic engineering.

In another aspect, the present application provides a cell, which may comprise one or more nucleic acid molecules of the present application and/or one or more vectors of the present application. In some embodiments, each kind of or each of the cells may comprise one or one kind of the nucleic acid molecules or the vectors of the present application. In some embodiments, each kind of or each of the cells may comprise multiple (e.g., two or more) or multiple kinds of (e.g., two or more kinds of) the nucleic acid molecules or the vectors of the present application. For example, the vector of the present application can be introduced into the cells, e.g., prokaryotic cells (e.g., bacterial cells), CHO cells, NS/0 cells, HEK293 cells, or other eukaryotic cells, such as plant-derived cells, fungi or yeast cells, and the like. The vector of the present application can be introduced into the cells by methods known in the art, such as electroporation, lipofectine transfection, lipofectamin transfection, and the like.

In another aspect, the present application provides a method for preparing the isolated antigen-binding protein, the chimeric antigen receptor or the polypeptide of the present application. The method may comprise culturing the cell of the present application under a condition enabling the expression of the isolated antigen-binding protein, the chimeric antigen receptor or the polypeptide, for example, by using an appropriate culture medium, an appropriate temperature, and a culture time, etc., which methods are known to those of ordinary skill in the art.

### Pharmaceutical composition, Application

In another aspect, the present application provides a pharmaceutical composition, which comprises the isolated antigen-binding protein, the immune cell, the polypeptide, the drug molecule, the nucleic acid molecule, the vector, the cell, and/or a pharmaceutically acceptable carrier.

For example, the pharmaceutically acceptable carrier may comprise a buffer, an antioxidant, a preservative, a low molecular weight polypeptide, a protein, a hydrophilic polymer, an amino acid, a carbohydrate, a chelating agent, a counterion, a metal complex and/or a nonionic surfactant.

In the present application, the pharmaceutical composition can be formulated for oral administration, intravenous administration, intramuscular administration, *in situ* administration at the tumor site, inhalation, rectal administration, vaginal administration, transdermal administration or administration via subcutaneous reservoir. The pharmaceutical composition can be used to inhibit the growth of tumors. For example, the pharmaceutical composition of the present application can inhibit or delay the development or progression of a disease, and/or alleviate and/or stabilize the disease state.

The pharmaceutical composition of the present application may comprise a prophylactically and/or therapeutically effective amount of the isolated antigen-binding protein. The prophylactically and/or therapeutically effective amount is a dose required to prevent and/or treat (at least partially treat) a disease or disorder and/or any complications thereof in a subject suffering from or at risk of developing the disease or disorder.

In another aspect, the present application provides a method that can be used for detecting or determining ROR1, which may comprise using the isolated antigen-binding protein, the chimeric antigen receptor or the polypeptide.

In another aspect, the present application provides a kit that can be used for detecting or determining ROR1 in a sample, which may comprise the isolated antigen-binding protein, the chimeric antigen receptor or the polypeptide.

In another aspect, the present application provides use of the isolated antigen-binding protein, the immune cell, the polypeptide, the drug molecule, the isolated nucleic acid molecule, the vector, the cell, and/or the pharmaceutical composition in the preparation of a drug for preventing and/or treating a disease and/or disorder.

In another aspect, the present application provides the isolated antigen-binding protein, the immune cell, the polypeptide, the drug molecule, the isolated nucleic acid molecule, the vector, the cell, and/or the pharmaceutical composition, which are used to prevent or treat a disease or disorder.

In another aspect, the present application provides a method for preventing or treating a disease, which comprises administering to a subject in need thereof the isolated antigen-binding protein, the immune cell, the polypeptide, the drug molecule, the isolated nucleic acid molecule, the vector, the cell, and/or the pharmaceutical composition of the present application.

In the present application, the disease and/or disorder can be caused or mediated by the abnormal expression of ROR1.

In the present application, the disease and/or disorder may comprise a tumor. For example, the tumor may comprise a solid tumor and/or a blood tumor. For example, the tumor may be an ROR1-positive tumor.

For example, the ROR1-positive tumor may be selected from: breast cancer, ovarian cancer, melanoma, pancreatic cancer, lung cancer, leukemia, lymphoma, and/or thyroid cancer.

Without intending to be limited by any theory, the following examples are only to illustrate various technical schemes of the present application, and not intended to limit the scope of the present application.

### EXAMPLES

### Example 1 Animal immunization

The experimental process was carried out in the following steps:
(1) A 2.5-year-old unimmunized female alpaca was immunized five times every two weeks with freshly prepared immunogen (see Table 1. Immunization schedule), where the immunogen was subpackaged in several portions (each portion for one immunization) and stored at -80°C;
(2) During the first immunization, 300 µg of immunogen was mixed with an equal volume of complete Freund's adjuvant (CFA) to make an emulsion; while for subsequent immunizations (from the 2nd to the 5th), 300 µg of immunogen was mixed with an equal volume of incomplete Freund's adjuvant (CFA) mixed adjuvant (IFA) to make an emulsion (all vaccination experiments were approved by the local Ethics Committee);
(3) Before each administration of immunogen (the 1st to the 5th), 5 ml of jugular vein blood sample was taken, and serum was recovered as the post-immunization IgG-containing sample for the antigen-specific antibody titer;
(4) Seven days after the last immunization, 50 ml of blood was collected, and peripheral blood mononuclear cells (PBMCs) were isolated from whole blood using Ficoll-Paque^{™} PLUS Media (GE Healthcare, USA) and lysed in Trizol for subsequent extraction of total RNA;
(5) The titers of serum before and after immunization were compared by ELISA to measure antigen-induced seroconversion, where one experiment was conducted on each diluted serum sample at each bleeding time point.

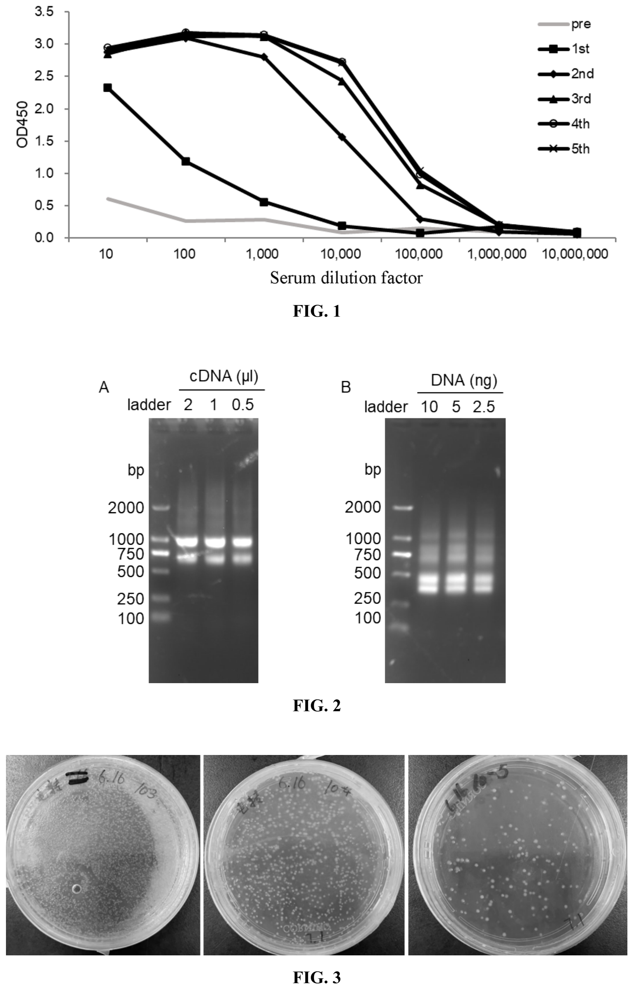

The experimental results are shown in FIG. 1, showing that the immune serum showed a significant response to the recombinant ROR1 protein, and the titers of ROR1-specific antibodies in the serum after the 3rd to the 5th immunizations all reached more than 1/100,000.

**Table 1. Immunization schedule**

| Day | Immunization | Experimental operations |
|---|---|---|
| 1 | 1st Immunization | Collect 5 ml of pre-immunization peripheral blood and store the serum |
| 15 | 2nd Immunization | Collect 5 ml of pre-immunization peripheral blood and store the serum |
| 42* | 3rd Immunization | Collect 5 ml of pre-immunization peripheral blood and store the serum |
| 56 | 4th Immunization | Collect 5 ml of pre-immunization peripheral blood and store the serum |
| 70 | 5th Immunization | Collect 5 ml of pre-immunization peripheral blood and store the serum |
| 77 | Blood sampling | Collect 50 ml of peripheral blood and store PBMC serum; Detect antibody titers of all samples |
| 86 | Collection | Analyze all sera, PBMCs, and titer data |

| | | |
|---|---|---|
| (*The 3rd immunization was postponed by two weeks due to the COVID-19 quarantine at the alpaca farm) | | |

### Example 2 Establishment of immunophage library

The experimental process was carried out in the following steps:
(1) After isolating PBMCs and extracting total RNA from 50 ml of peripheral blood, cDNA was prepared from commercially available reagents using RNA as a template, with the preparation system shown in Table 2;
(2) After amplifying VHH sequences from the cDNA library and transforming them into TGI cells, the library size was calculated based on the number of colonies in a series of dilutions of the electroporated E. coli cell suspension on a 90 mm plate;
(3) Colony sequencing and bioinformatics analysis were conducted.

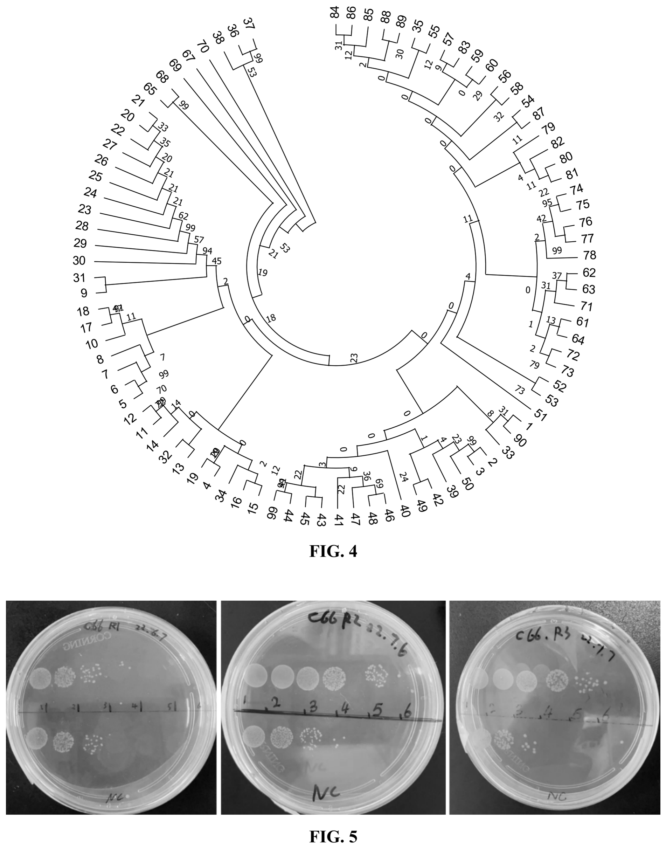

The experimental results show that two groups of different amplicons were generated from the first PCR, with molecular weights of approximately 0.7 kb and 0.9 kb (FIG. 2A); the 0.7 kb amplicon was used as the template to amplify VHH, obtaining a DNA fragment with a fragment size of about 400bp (FIG. 2B), and the library size after amplification was 1.4×10⁸ (FIG. 3). Phylogenetic analysis was conducted on 90 sequences in the library. 96 single colonies were randomly selected for colony sequencing. The proportion of phage with VHH correctly inserted was 93.8% (90/96), and the proportion of specific VHH sequences was 96.7% (87/90) (FIG. 4).

**Table 2. Production of total RNA, cDNA, and PCR products**

| Sample | Depth | Volume (µl) | Total amount (µg) |
|---|---|---|---|
| Total RNA | 1.02 µg/µl | 240 | 244.8 |
| cDNA | NA | 2500 | NA |
| Products of the first round of PCR | 44.6 ng/µl | 1650 | 73.59 |
| Products of the second round of PCR | 182.4 ng/µl | 480 | 87.55 |

### Example 3 Screening of antigen specific antigen-binding protein

Three consecutive rounds of screening were conducted by monitoring the number of colonies growing on the plate, obtaining the specific antigen-binding protein of ROR1 (FIG. 5). After each round of screening, the number of colonies in the ROR1-coated group increased, indicating that the ROR1-specific antigen-binding proteins were enriched. R1, R2 and R3 represent the screening results of the first round, the second round and the third round, respectively, and NC represents the negative control.

### Example 4 Identification and verification of antigen-binding protein

(1) The specific antigen-binding proteins after three rounds of screening were verified by ELISA identification using periplasmic extracts to obtain antigen-positive clones;
(2) The DNA sequences of positive clones were then obtained through colony sequencing to identify the specific sequences;
(3) The specific sequences were reconstituted for production and purification, and the purity was detected to determine whether it met the requirements for subsequent detection;
(4) The affinity and specificity of the antigen-binding protein for the ROR1 protein were further detected.

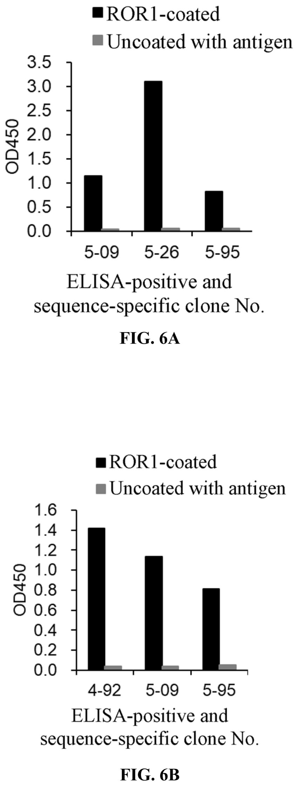

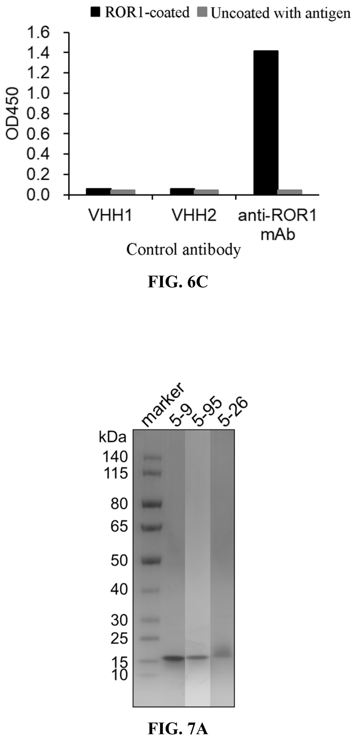

The experimental results show that the OD450 values of 5-26 (with the VHH sequence as set forth in SEQ ID NO: 8) and 4-92 (with the VHH sequence as set forth in SEQ ID NO: 25) were all higher than those of 5-09 (with the VHH sequence as set forth in SEQ ID NO: 11) and 5-95 (with the VHH sequence as set forth in SEQ ID NO: 12) (FIG. 6A and FIG. 6B). Where, 5-26 comprises a CDR1 as set forth in SEQ ID NO: 1, a CDR2 as set forth in SEQ ID NO: 2, and a CDR3 as set forth in SEQ ID NO: 3; 4-92 comprises a CDR1 as set forth in SEQ ID NO: 19, a CDR2 as set forth in SEQ ID NO: 20, and a CDR3 as set forth in SEQ ID NO: 21. Two non-specific VHHs (VHH1: the sequence is as set forth in SEQ ID NO: 9; VHH2: the sequence is as set forth in SEQ ID NO: 10) did not bind to the recombinant ROR1protein, while the binding of anti-ROR1 mAb to the recombinant ROR1 protein was good (FIG. 6C). The experimental results further indicate that, 5-26, 4-92, 5-09, and 5-95 specifically bound to the recombinant ROR1 protein, but the binding ability of 5-26 and 4-92 was obviously superior to that of the other two groups.

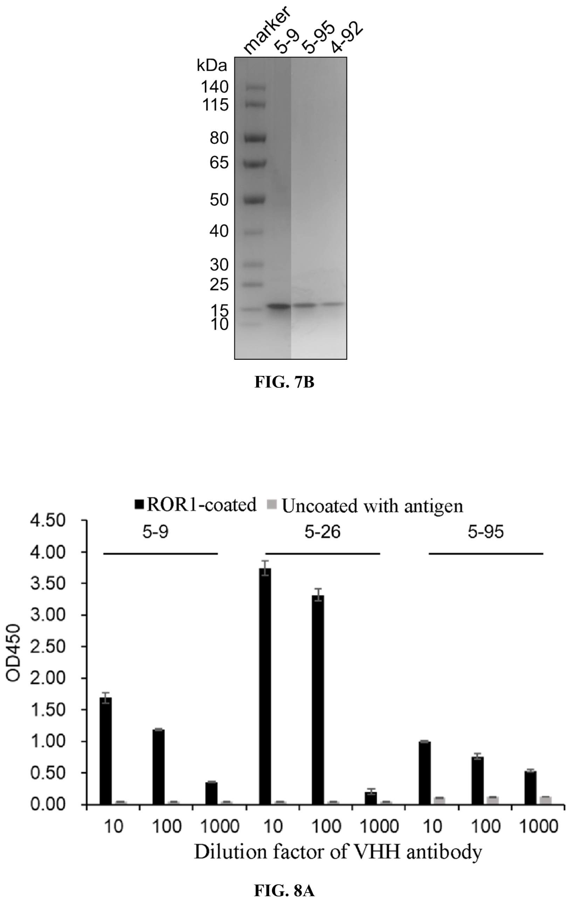

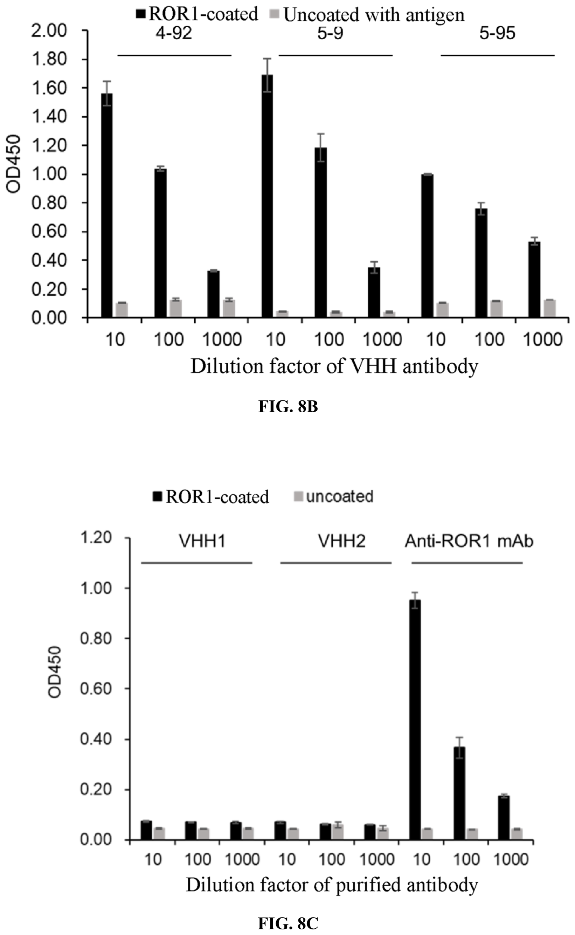

The SDS-PAGE results after purification show that, the purity of the antigen-binding protein (each about 0.5 mg) exceeded 90%, satisfying the requirements for subsequent function test (FIGs. 7A-7B), the purified antigen-binding proteins 5-26, 4-92, 5-9, and 5-95 specifically bound to the recombinant ROR1 protein (FIGs. 8A-8B), the negative control VHH1 and VHH2 did not bind to the ROR1 protein, while the positive control bound to the ROR1 protein (FIG. 8C). The experimental results indicate that, compared with 5-9 and 5-95, 5-26 and 4-92 could bind to the ROR1 protein at a relatively high concentration under multiple dilution factors, and have good specificity.

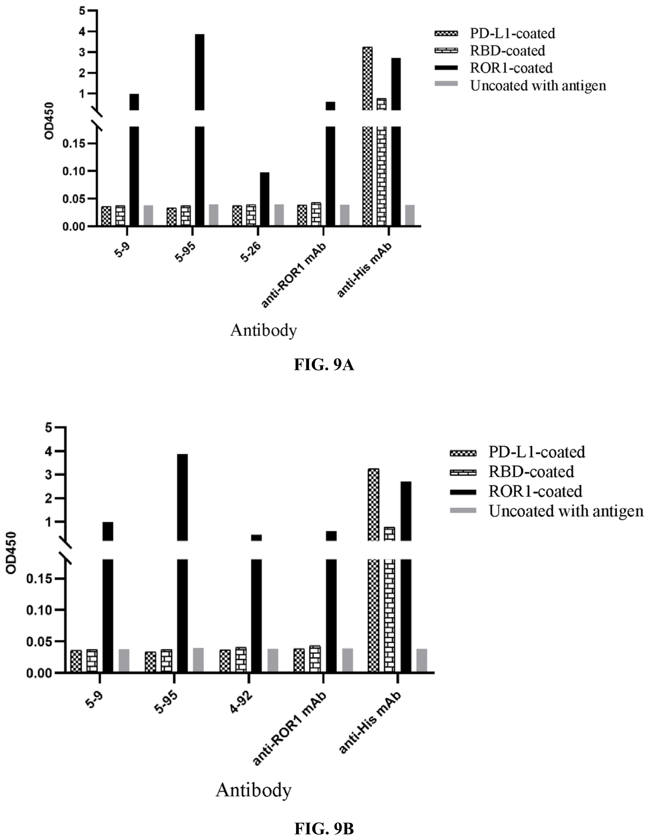

Meanwhile, the specificity evaluation shows that, the purified antigen-binding proteins 5-26, 4-92, 5-9, and 5-95 only bound to the recombinant ROR1 protein, but did not bind to the other two his-tagged proteins (PD-L1 and RBD), indicating that the selected antigen-binding proteins did not identify other tags. Anti-ROR1 mAb (positive control) only bound to the recombinant ROR1 protein. Anti-his mAb (Sinobiological, # 105327-MM02T-H) could bind to all the three his-tagged proteins (PD-L1, RBD, ROR1) (FIGs. 9A-9B). The concentration of the purified antigen-binding proteins used in the experiments was all 3 µg/ml, one his tag and one HA tag were comprised at the C-terminus of the proteins, and the secondary antibody used for identification was HRP conjugated anti-HA tag antibody (Abcam, #ab1190). The experimental results indicate that, 5-26 and 4-92 have good specificity for the ROR1 protein, and substantially do not bind to PD-L1 and RBD.

anti-ROR1 mAb: ROR1 mouse monoclonal antibody, from Proteintech (catalog No.: 66923-1-Ig), with a molecular weight of 130 kDa, can be used in applications such as FC, IF, IHC, WB, and ELISA.

### Example 5 Detection of ability of antigen-binding protein to bind to ROR1-expressing cell lines

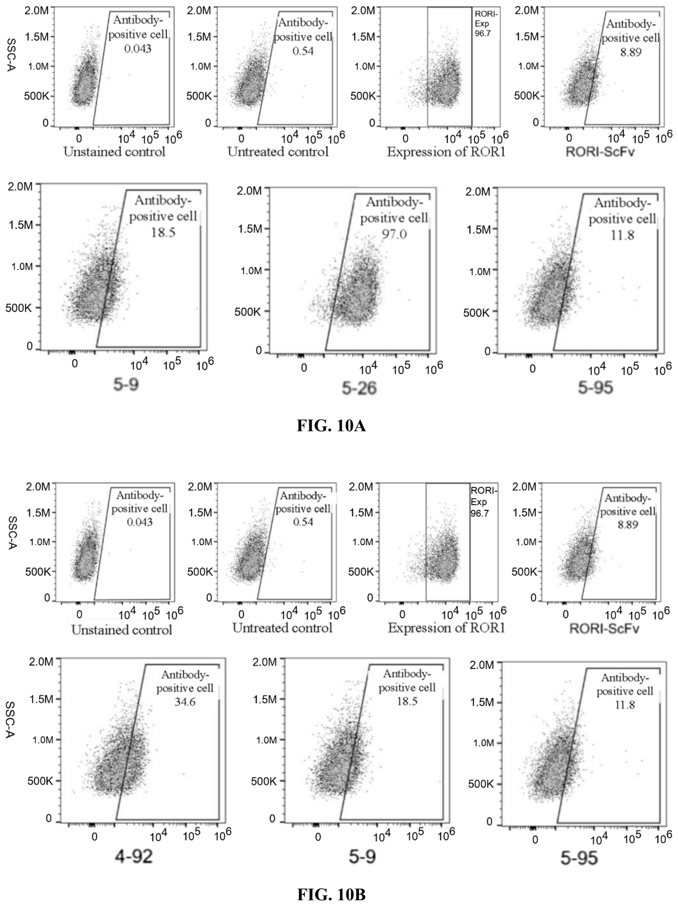

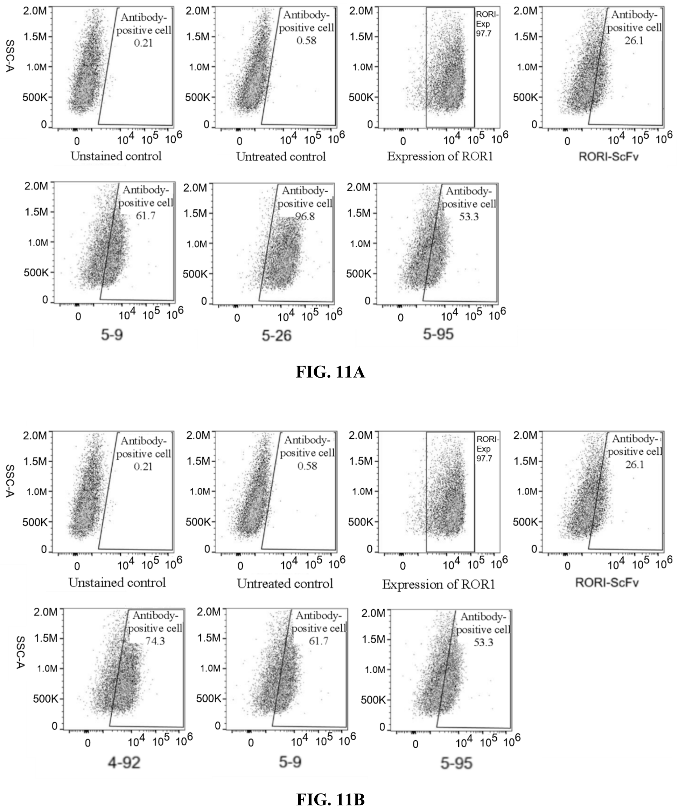

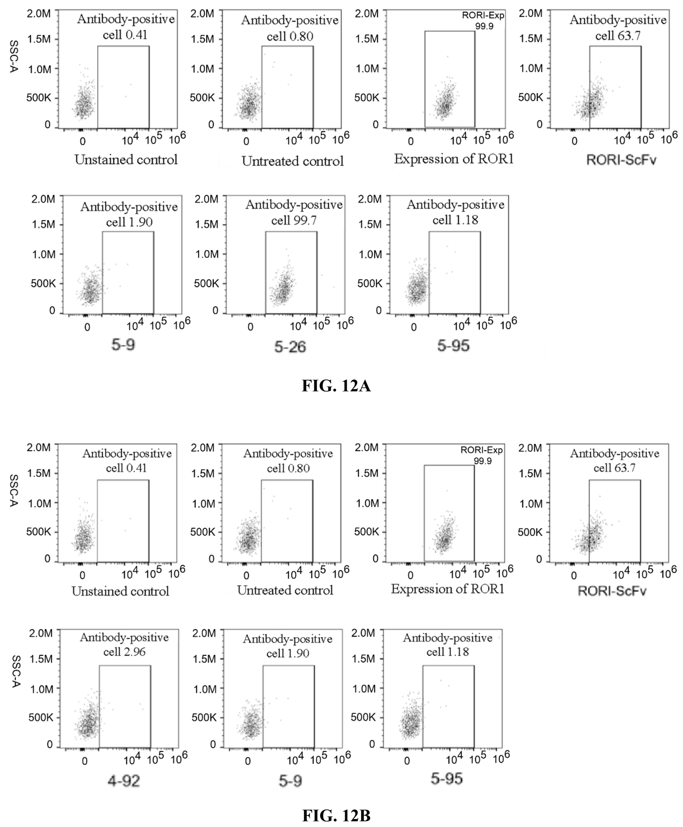

ROR1 was expressed in A549, MB231, and JeKo-1 cells at 1.5-2.0×10⁵ cells per well. The antigen-binding protein was co-incubated with the cells at a concentration of 1 µg/ml at 4°C for 30 minutes and stained with anti-HA APC. The results of flow cytometry show that, in A549 cells, the proportion of binding cells was 97.0% for 5-26, 18.5% for 5-9, and 11.8% for 5-95 (FIG. 10A), and the proportion of binding cells reached 34.6% for 4-92, 18.5% for 5-9, and 11.8% for 5-95 (FIG. 10B). In MB231 cells, the proportion of binding cells reached 96.8% for 5-26, 61.7% for 5-9, and 53.3% for 5-95 (FIG. 11A), and the proportion of binding cells reached 74.3% for 4-92, 61.7% for 5-9, and 53.3% for 5-95 (FIG. 11B). In JeKo-1 cells, the proportion of binding cells reached 99.7% for 5-26, 1.90% for 5-9, and 1.18% for 5-95 (FIG. 12A), and the proportion of binding cells reached 2.96% for 4-92, 1.90% for 5-9, and 1.18% for 5-95 (FIG. 12B). The experimental results indicate that, compared with 5-9 and 5-95, 5-26 and 4-92 have stronger ability of binding to ROR1-expressing cell lines.

### Example 6 Detection of ability of humanized antigen-binding protein to bind to ROR1-expressing cell lines

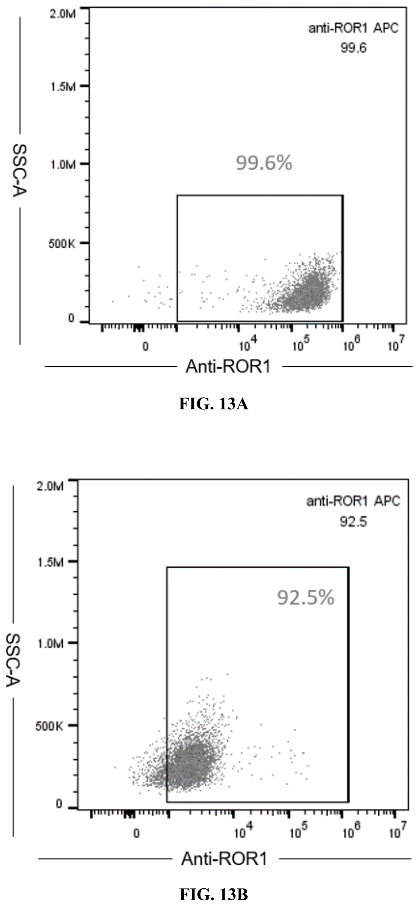

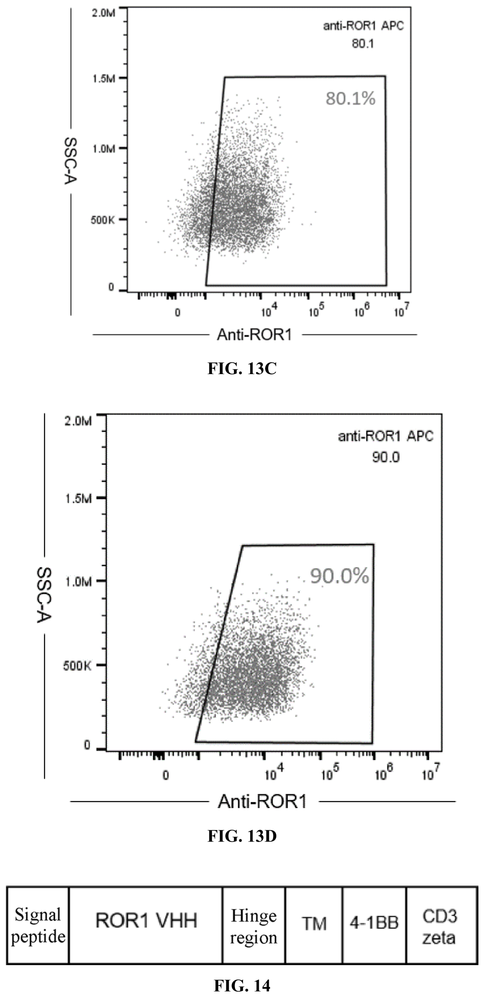

The sequence of 5-26 was humanized to obtain the amino acid sequence of hu5-26. For the hu5-26, the amino acid sequence of CDR1 is as set forth in SEQ ID NO: 1,the amino acid sequence of CDR2 is as set forth in SEQ ID NO: 2, the amino acid sequence of CDR3 is as set forth in SEQ ID NO: 13, the amino acid sequence of FR1 is as set forth in SEQ ID NO: 14, the amino acid sequence of FR2 is as set forth in SEQ ID NO: 15, the amino acid sequence of FR3 is as set forth in SEQ ID NO: 16, the amino acid sequence of FR4 is as set forth in SEQ ID NO: 17, and the amino acid sequence of VHH is as set forth in SEQ ID NO: 18. hu5-26 was linked to IgG Fc to obtain a hu5-26 single-domain antibody. The ability of hu5-26 to bind to different ROR1-expressing cell lines (MECs) was detected by using the method in Example 5. The experimental results are shown in FIGs. 13A-13D. The experimental results show that: in MEC cells, the proportion of hu5-26-binding cells reached 99.6% (FIG. 13A); in JeKo-1 cells, the proportion of hu5-26-binding cells reached 92.5% (FIG. 13B); in A549 cells, the proportion of hu5-26-binding cells reached 80.1% (FIG. 13C); and in MB231 cells, the proportion of hu5-26-binding cells reached 90.0% (FIG. 13D).

In the present application, the ROR1 binding ability of hu5-26 is stronger than that of other humanized antibodies, and has stronger affinity for almost all the ROR1-positive cells.

### Example 7 Preparation and expression of ROR1 nano-chimeric antigen receptor

### Culture of cells

HEK293T, MEC1, and MDA-MB-231 cell lines are available from ATCC, USA. MEC1-ROR1 cell lines are internally generated stable ROR1-expressing strains. HEK293T cell lines were maintained in a complete medium (DMEM containing 10% heat-inactivated FBS, 100 U/mL penicillin/streptomycin, and 2 mM L-glutamine). MDA-MB-231, MEC1 and MEC1-ROR1 cell lines were maintained in a complete medium (IMDM containing 10% heat-inactivated FBS, 100 U/mL penicillin/streptomycin, and 2 mM L-glutamine). PBMCs were isolated from the whole blood of healthy donors using Ficoll-Paque and aliquoted into 1 mL of the sample at a concentration of 2 × 10⁷ cells/mL. The sample was cryopreserved in a liquid nitrogen tank, with the medium being thermally inactivated FBS supplemented with 10% DMSO (vol/vol).

### Construction of clones

All related gene structures are constructed and synthesized by GeneWiz. The ROR1 nano-chimeric antigen receptor adopts the structure of the second-generation CAR (FIG. 14). The gene structure encoding hu5-26 ROR1 VHH CAR (hereinafter referred to as ROR1VHH CAR) (for the ROR1 VHH, CDR1 is as set forth in SEQ ID NO: 1, CDR2 is as set forth in SEQ ID NO: 2, CDR3 is as set forth in SEQ ID NO: 13, VHH is as set forth in SEQ ID NO: 18, the amino acid sequence of the signal peptide is as set forth in SEQ ID NO: 26, the amino acid sequence of the hinge region is as set forth in SEQ ID NO: 27, the amino acid sequence of the transmembrane domain is as set forth in SEQ ID NO: 28, the amino acid sequence of the costimulatory domain is as set forth in SEQ ID NO: 29, the amino acid sequence of the intracellular signaling domain is as set forth in SEQ ID NO: 30) was inserted into the pALD expression plasmid through BamHI and SalI cloning sites. After verification by double enzyme digestion and sequencing, the expression plasmid was massively amplified and used for the packaging of lentiviruses.

### Preparation of lentiviruses

All lentiviruses were prepared from HKE293T. After washing the freshly resuspended HEK293T cells in PBS buffer, the cells were seeded in 10-cm culture dishes with a cell density of 90%-95% of the dish area. The mixed lentivirus packaging plasmid and transfection plasmid were mixed with the transfection agent Lipofectamine-3000 and then transiently transfected into the seeded HEK293T cells. After 48 hours of culture, the supernatant was collected and filtered through a 0.45-µm filter, then aliquoted into 1-ml tubes and stored in a refrigerator at -80°C.

### Activation and transduction of T cells

In order to transduce the lentiviruses encoding the corresponding structure into PBMCs, the cryopreserved PBMCs were resuscitated and added into a 24-well plate for activation with the addition of CD3 and CD28 coupled magnetic beads and IL2 (500 IU/ml) for 24-72 hours. After distributing the activated PBMC cells into multiple wells of the 24-well plate, the corresponding lentiviruses and polybrene (8 g/ml) were added. The cells were centrifuged in a tabletop centrifuge at 2000 g for 2 hours and then transferred into an incubator at 37°C and 5% CO₂ for culture. For the control group, the lentiviruses were replaced with complete culture medium, and the remaining steps were the same. The transfected cells were further cultured in X-VIVO-15 cell culture medium containing IL2 (500 IU/ml) for 48 hours for subsequent tests and analysis.

### Flow cytometry assay

Flow cytometry assay was performed using a flow cytometer (Beckman Coulter) in semiautomatic or plate mode, and the data were analyzed using FlowJo software. The cells were washed once with FACS buffer (containing 0.5% BSA, 0.1% NaN₃, 2 mM EDTA, and PBS at pH 7.0), resuspended to 1~5 × 10⁶ cells/mL, and placed on ice before staining. Into the cells were added antibodies with fluorophores for staining, and incubated in the dark in a refrigerator at 4°C for 40 minutes, and 7-AAD was then added to continue staining for 5 minutes. After staining was completed, a large amount of FACS buffer was added to wash away unbound antibodies. Centrifugation was performed at 300×g for 3 minutes to remove the supernatant. After washing and suspending with FACS buffer, the cells were analyzed by flow cytometry.

### Enzyme-linked immunosorbent assay

The supernatant after co-culture was carefully aspirated after simple centrifugation, and can be used in the enzyme-linked immunosorbent assay after appropriate dilution. According to the manufacturer's instructions, the secretion levels of cytokines in the culture solution were measured using ELISA MAX^{™} Deluxe Set Human IFN-γ kit (Biolegend; 430104) and ELISA MAX^{™} Standard Set Human IL-2 (Biolegend;431801) kit.

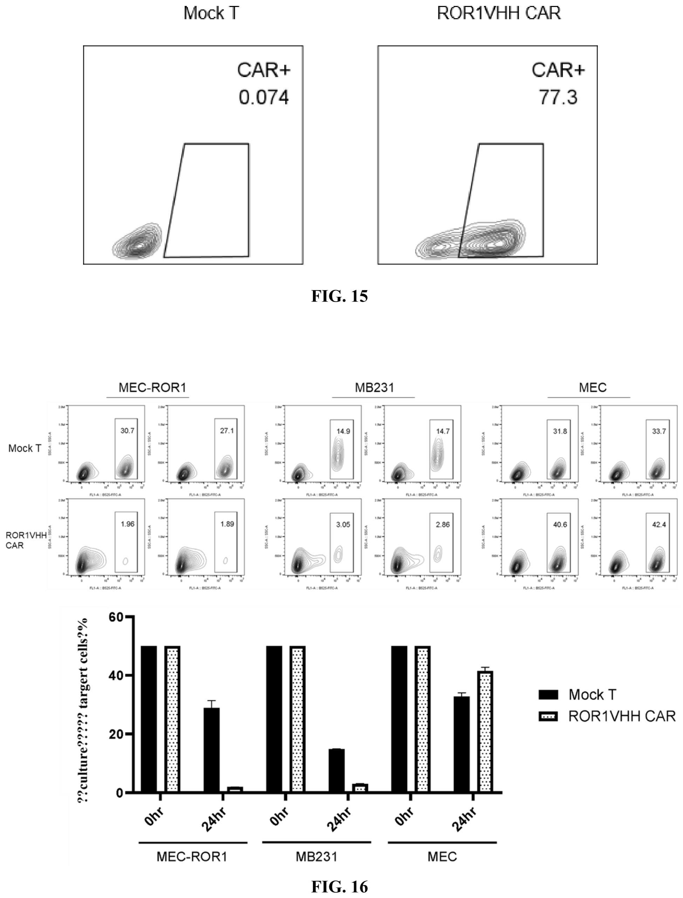

The structure containing hu5-26 ROR1 CAR was successfully cloned and packaged into lentiviruses. As shown in FIG. 15, after 72 hours of lentivirus infection, the activated human PBMCs were detected to have a high level of expression of the CAR structure and could bind to the PE-labeled ROR1 protein.

### Example 8 Experimental verification of In vitro function of ROR1 nano-chimeric antigen receptor

### Tumor cell lines

The tumor cells MEC1 (B-cell chronic lymphocytic leukemia) were modified to produce a cell line stably expressing the ROR1 antigen. The MDA-MB-231 cell line has already expressed the ROR1 antigen by itself. These three tumor cell lines were transfected to express GFP and were used in subsequent experiments.

### CAR-T tumor short-acting killing experiment (flow cytometry assay)

The above structure (ROR1VHH CAR) was transduced into PBMC cells and cultured for 72 hours, and then the expression of CAR was detected by flow cytometry. The effector cells and target cells were precisely counted respectively, then resuspended in 200 µl of cell culture medium at an effector-to-target ratio of 1:1 and a cell density of 1=5 × 10⁴, and seeded in a 96-well plate for culture. 24 hours later, the 96-well plate was taken out, centrifuged, and the supernatant was used to determine the secretion of γ-interferon and IL2 by enzyme-linked immunosorbent assay.

Meanwhile, the co-cultured cells were washed with PBS and resuspended, and then stained with 7-AAD in the dark for 5 minutes, and the proportion of target cells in the surviving cells was detected by flow cytometry.

### CAR-T tumor long-acting killing experiment (Incucyte real-time imaging detection)

The above structure (ROR1VHH CAR) was transduced into PBMC cells and cultured for 72 hours, and then the expression of CAR was detected by flow cytometry. The effector cells and target cells were precisely counted respectively, then resuspended in 200 µl of cell culture medium at an effector-to-target ratio of 1:1 and a cell density of 1=1 × 10⁴, and seeded in a flat-bottomed 96-well plate for culture. The 96-well plate was placed in IncuCyte for real-time imaging to monitor the 48-hour growth curve of GFP-labeled target cells.

In the short-acting killing assay by flow cytometry, since the target cells are labeled with GFP, the target cells that are still alive in the entire co-culture system can be counted. As shown in FIG. 16, after 24 hours, the ROR1VHH CAR group showed a strong killing ability against ROR1-expressing target cells, with the proportion of target cells decreasing from 50% at 0 h to 1.925% (MEC-ROR1) and 2.955% (MB231), respectively. However, for the target cells that did not express ROR1, there was no significant difference from the control group, showing good specificity.

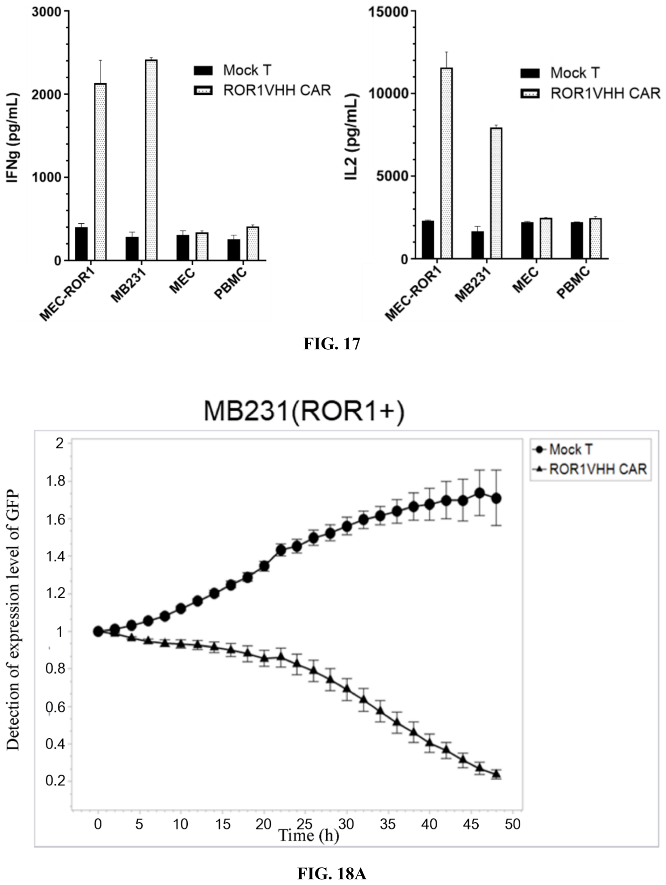

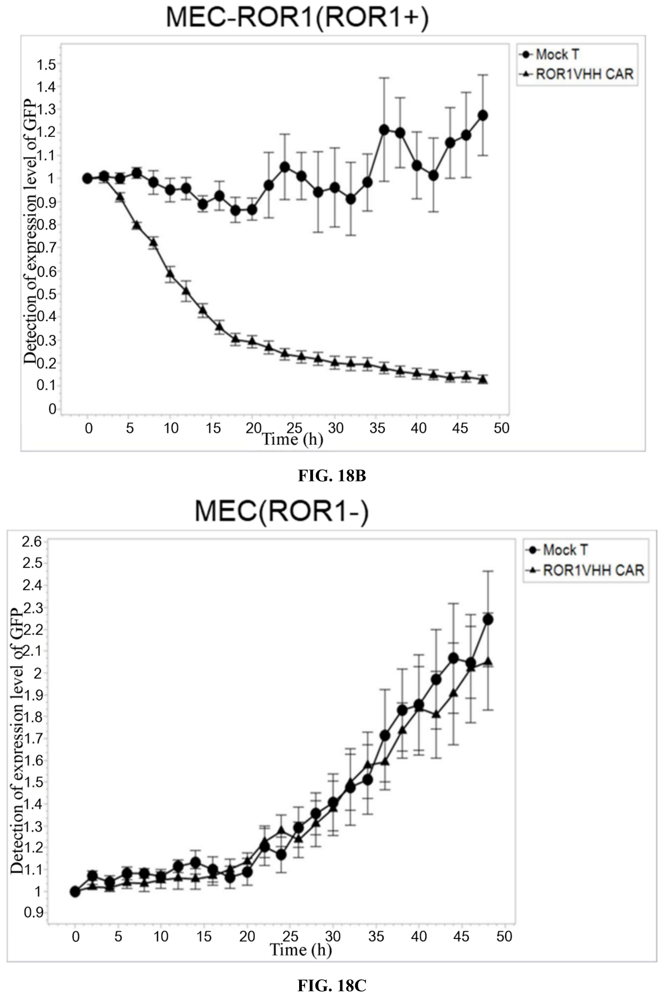

Meanwhile, during the process of killing ROR1-positive target cells, higher level of secretion of γ-interferon and IL2 could be detected in the hu5-26 ROR1VHH CAR group (FIG. 17). As shown in FIGs. 18A-18C, in the IncuCyte real-time imaging experiment, the ROR1 VHH CAR group could effectively inhibit the *in vitro* proliferation of ROR1-positive tumor cells and could substantially clear the target cells within 48 hours (MB231 killing 80%; MEC-ROR killing 90%). Likewise, this tumor-killing ability also showed good specificity and had excellent safety in practical applications.

The foregoing detailed description is provided by way of explanation and examples and is not intended to limit the scope of the appended claims. Various changes of the embodiments currently set forth in this application are obvious to those of ordinary skills in the art and are reserved within the scope of the appended claims and their equivalents.

## Claims

1. An isolated antigen-binding protein, being capable of binding to a receptor tyrosine kinase-like orphan receptor 1 (ROR1), said isolated antigen protein comprises CDR1, CDR2, and CDR3, and said CDR1, CDR2, and CDR3 comprise any one group of amino acid sequences selected from:
1) the amino acid sequence of said CDR1 is as set forth in SEQ ID NO: 1, the amino acid sequence of said CDR2 is as set forth in SEQ ID NO: 2, and the amino acid sequence of said CDR3 is as set forth in SEQ ID NO: 3;
2) the amino acid sequence of said CDR1 is as set forth in SEQ ID NO: 1, the amino acid sequence of said CDR2 is as set forth in SEQ ID NO: 2, and the amino acid sequence of said CDR3 is as set forth in SEQ ID NO: 13;
3) the amino acid sequence of said CDR1 is as set forth in SEQ ID NO: 19, the amino acid sequence of said CDR2 is as set forth in SEQ ID NO: 20, and the amino acid sequence of said CDR3 is as set forth in SEQ ID NO: 21,
said isolated antigen-binding protein is a VHH.

2. The isolated antigen-binding protein of claim 1, wherein the amino acid sequence of said VHH is as set forth in SEQ ID NO: 8, SEQ ID NO: 18 or SEQ ID NO: 25.

3. The isolated antigen-binding protein of any one of claims 1-2, comprising an antibody or an antigen-binding fragment thereof.

4. The isolated antigen-binding protein of any one of claims 1-3, wherein said antibody is selected from a group consisting of: a monoclonal antibody, a polyclonal antibody, a chimeric antibody, a humanized antibody, and a fully human antibody.

5. The isolated antigen-binding protein of any one of claims 1-4, comprising at least one VHH fragment.

6. The isolated antigen-binding protein of any one of claims 1-5, comprising two VHH fragments.

7. The isolated antigen-binding protein of any one of claims 1-6, further comprising an Fc region.

8. The isolated antigen-binding protein of claim 7, wherein said Fc region is derived from an IgG Fc region.

9. The isolated antigen-binding protein of claim 8, wherein said Fc region is derived from a human IgG Fc region.

10. The isolated antigen-binding protein of any one of claims 1-9, having at least one of the following properties:
capable of specifically binding to an ROR1; and
capable of binding to an ROR1 expressed on a cell surface.

11. A chimeric antigen receptor, comprising the isolated antigen-binding protein of any one of claims 1-10.

12. The chimeric antigen receptor of claim 11, further comprising a transmembrane domain, a costimulatory domain, and an intracellular signaling domain.

13. The chimeric antigen receptor of claim 12, wherein said transmembrane domain comprises a transmembrane domain derived from a protein selected from a group consisting of: CD8, CD28, 4-1BB, CD4, CD27, CD7, PD-1, TRAC, TRBC, CD3ε, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154 or SLAM.

14. The chimeric antigen receptor of claim 13, wherein said transmembrane domain is derived from a transmembrane domain of CD28, and said transmembrane domain comprises the amino acid sequence as set forth in SEQ ID NO: 28.

15. The chimeric antigen receptor of any one of claims 12-14, wherein said costimulatory domain comprises a costimulatory domain derived from one or more proteins selected from a group consisting of: CD28, 4-1BB, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, a ligand of CD83, CD40, and MyD88.

16. The chimeric antigen receptor of claim 15, wherein said costimulatory domain is derived from a 4-1BB costimulatory domain, and said costimulatory domain comprises the amino acid sequence as set forth in SEQ ID NO: 29.

17. The chimeric antigen receptor of any one of claims 12-16, wherein said intracellular signaling domain comprises an intracellular signaling region derived from one or more proteins selected from a group consisting of: CD3ζ, CD3δ, CD3γ, CD3ε, CD79a, CD79b, FcεRIγ, FcεRIβ, FcγRIIa, bovine leukemia virus gp30, Epstein-Barr virus (EBV) LMP2A, simian immunodeficiency virus PBj14 Nef, Kaposi's sarcoma-associated herpes virus (HSKV), DAP10, DAP-12, and a domain containing at least one ITAM.

18. The chimeric antigen receptor of claim 17, wherein said intracellular signaling domain is a signaling domain derived from CD3ζ, and said intracellular signaling domain comprises the amino acid sequence as set forth in SEQ ID NO: 30.

19. The chimeric antigen receptor of any one of claims 11-18, further comprising a hinge region.

20. The chimeric antigen receptor of claim 19, wherein said hinge region is a hinge region derived from CD28.

21. The chimeric antigen receptor of claim 20, wherein said hinge region comprises the amino acid sequence as set forth in SEQ ID NO: 27.

22. The chimeric antigen receptor of any one of claims 11-21, comprising the amino acid sequence as set forth in SEQ ID NO: 31.

23. An immune cell, comprising the chimeric antigen receptor of any one of claims 11-22.

24. The immune cell of claim 23, being selected from: a T cell, an NK cell, an iNKT cell, a CIK cell or a γδT cell.

25. A drug molecule, comprising the isolated antigen-binding protein of any one of claims 1-10, or the chimeric antigen receptor of any one of claims 11-22.

26. A nucleic acid molecule, encoding the isolated antigen-binding protein of any one of claims 1-10, or the chimeric antigen receptor of any one of claims 11-22.

27. A vector, comprising the nucleic acid molecule of claim 26.

28. A host cell, comprising the nucleic acid molecule of claim 26, and/or the vector of claim 27.

29. A pharmaceutical composition, comprising the antigen-binding protein of any one of claims 1-10, the immune cell of any one of claims 23-24, the drug molecule of claim 25, the nucleic acid molecule of claim 26, the vector of claim 27, and/or the host cell of claim 28, as well as optionally a pharmaceutically acceptable carrier.

30. A kit, comprising the isolated antigen-binding protein of any one of claims 1-10 or the chimeric antigen receptor of any one of claims 11-22, the kit being capable of detecting the presence and/or content of ROR1 in a sample.

31. Use of the isolated antigen-binding protein of any one of claims 1-10, the chimeric antigen receptor of any one of claims 11-22, the immune cell of any one of claims 23-24, the drug molecule of claim 25, the nucleic acid molecule of claim 26, the vector of claim 27, the host cell of claim 28, the pharmaceutical composition of claim 29 in the preparation of a drug for preventing and/or treating an ROR1-related disease and/or disorder.

32. The use of claim 31, wherein said disease and/or disorder comprises a tumor.

33. The use of claim 32, wherein said tumor is a solid tumor and/or a blood tumor.

34. The use of any one of claims 32-33, wherein said tumor is an ROR1-positive tumor.
